# EUROPÄISCHE PATENTSCHRIFT

(11) **EP 3 770 547 B1**
(45) Veröffentlichungstag und Bekanntmachung des Hinweises auf die Patenterteilung: **23.02.2022**
(21) Anmeldenummer: 19188508.6
(22) Anmeldetag: 26.07.2019
(51) Int. Cl.: G01B 11/25, G01N 21/84, G01N 33/38

(54) **STEINANALYSEVORRICHTUNG UND VERFAHREN ZUR BEWERTUNG VON STEINEN**
STONE ANALYSIS DEVICE AND METHOD FOR EVALUATING STONES
DISPOSITIF D'ANALYSE DE PIERRE ET PROCÉDÉ D'ÉVALUATION DE PIERRES

(43) Veröffentlichungstag der Anmeldung: 27.01.2021
(73) Patentinhaber: R&W Industrieautomation GmbH, 57627 Hachenburg (DE)
(72) Erfinder: Rahn, Uwe, 57642 Alpenrod (DE)
(74) Vertreter: Hannke, Christian

(56) Entgegenhaltungen:
- EP-A1- 0 657 260
- DE-U1- 29 702 402
- JP-A- H02 285 235
- JP-A- H07 128 029
- US-A1- 2012 304 763
- US-A1- 2017 249 727
- Gmbh Ibea ET AL: "We keep an eye on your quality", , 26 April 2017 (2017-04-26), pages 1-4, XP055794411, Retrieved from the Internet: URL:https://www.ibea.de/files/ibea_bt_mast er_deutsch.pdf [retrieved on 2021-04-12]
- Ibea GmbH: "BT-Master - 100% Quality control for your Bricks or Tiles", , 20 April 2017 (2017-04-20), page 1, XP054981666, Retrieved from the Internet: URL:https://www.youtube.com/watch?v=z27R9Q 575vk [retrieved on 2021-04-12]

## Beschreibung

Die vorliegende Erfindung betrifft eine Steinanalysevorrichtung zur Bewertung von Steinen, die mindestens zwei Scannereinheiten zum Scannen mindestens einer physikalischen Eigenschaft der Steine aufweist. Die vorliegende Erfindung betrifft auch ein Steinanalyseverfahren zur Bewertung von Steinen und Ermitteln eines Abstandes einer Förderfläche relativ zu der oberen Fläche des auf der Förderfläche befindlichen Steins.

Bei der Produktion von Betonsteinen und anderen künstlich hergestellten Steinen, oder in Form gebrachten Natursteinen ist es nötig, die Steine nach der Verarbeitung auf ihre Eigenschaften zu prüfen. Neben der Farbgebung sind insbesondere die Abmessungen und Oberflächen der Steine von großer Bedeutung. Die Betonsteine werden üblicherweise auf einer Produktionsplatte produziert, oder nach der Produktion auf eine solche Platte gelegt. Diese Produktionsplatte wird dann mit Hilfe von Fördersystemen transportiert und mit den darauf befindlichen Steinen durch eine Qualitätsüberwachungsanlage geleitet, die sich üblicherweise dicht hinter der Fertigungsanlage befindet. Dadurch ist ein direktes Feedback der Produktionsqualität sichergestellt.

Im Zuge dessen werden die physikalischen Eigenschaften der Steine analysiert. Während des Transports der Steine, wird beispielsweise das Höhenprofil der Produktionsplatte und der darauf befindlichen Betonsteine mittels Lasersensoren aufgezeichnet. Bei einer Oberflächenanalyse werden die Betonsteine auf unerwünschte Erhöhungen und Vertiefungen in der Oberfläche untersucht. Diese können bei der Produktion entstehen, falls z.B. zu feuchter Beton an den Formstempeln der Betonsteinmaschine hängen bleibt. Weiterhin kann die Oberflächenstruktur auf Unregelmäßigkeiten überprüft werden, die z.B. durch fehlerhafte Betonmischungen entstehen können. Es können auch Kantenausbrüche erkannt werden die beispielsweise beim Entformen von Betonsteinen entstehen können.

Nachdem die Produktionsplatte die Laser- und andere Sensoren passiert hat, erfolgt die Auswertung der aufgezeichneten Qualitätsdaten und damit auch beispielsweise der Höhenbestimmung der Betonsteine. Bisher wurde die Höhenmessung der Seine mit Hilfe von Laserdistanzsensoren durchgeführt. Diese Sensoren messen den Abstand vom Messobjekt, also den Steinen, und der Produktionsplatte zum Sensor mit einem Laserstrahl und einem integrierten Lichtsensor mit Hilfe des Triangulationsverfahrens. Dafür werden üblicherweise nur Punktlaserverwendet.

Problematisch dabei ist, dass entweder je Punktlaser nur eine Reihe von Steinen auf der Produktionsplatte vermessen werden kann, oder der reflektierte, also zu messende Strahl, bei zu hohen Messobjekten verdeckt, und demnach nicht erfasst bzw. der Abstand zur Produktionsplatte nicht gemessen werden kann. Dieses Problem kann auch bei Linienscannern auftreten. Hinzu kommt, dass sich die Vermessung der Oberfläche der Produktionsplatte besonders schwierig gestaltet, wenn die Produktionsplatte uneben ist, oder Beschädigungen in Form von beispielsweise Rissen aufweist. Derartige Unebenheiten sind besonders mit Punkt- und Linienscannern schwer oder gar nicht erfassbar, wenn der Abstand der Messobjekte derart gering ist, dass der Mess-Laserstrahl zwar die Oberfläche der Produktionsplatte erreicht, der reflektierte Strahl, welcher detektiert werden soll allerdings von Messobjekten verdeckt wird und dadurch den Detektor nicht erreicht. Für eine bessere Höhenmessung von vielen Steinen auf einer unebenen Produktionsplatte werden daher sehr viele Punktlaser benötigt, die aufeinander abgestimmt werden müssen und somit sehr hohe Kosten anfallen. Beispielsweise müsste jeweils ein Punktlaser pro Reihe von Messobjekten eingesetzt werden, wodurch bei 10 Reihen schon 10 Punktlaser notwendig wären.

Die Aufgabe der vorliegenden Erfindung ist es daher eine Steinanalysevorrichtung bereitzustellen, die eine exakte Höhenmessung der Steine bei gleichzeitig geringem technischem und finanziellem Aufwand ermöglicht.

Diese Aufgabe wird gemäß den Merkmalen des Anspruchs 1 gelöst, wonach eine Steinanalysevorrichtung zur Bewertung von Steinen, insbesondere Betonsteinen, angeordnet an einer Fördereinrichtung zum Fördern der Steinemit einer Förderfläche zum Auflegen der Steine, welche sich in einer Längsrichtung und einer Querrichtung (die bevorzugt senkrecht zueinander stehen) erstreckt. Die Steinanalysevorrichtung weist weiterhin mindestens eine erste Scannereinheit zum Scannen mindestens einer physikalischen Eigenschaft der Steine, eine Auswerteeinrichtung zum Auswerten eines von der Scannereinheit ausgegebenen Signals, eine Steuereinrichtung und eine Anzeigeeinrichtung auf. Die Scannereinheit ist von der Förderfläche in einer Vertikalrichtung, senkrecht zur Längsrichtung und Querrichtung beabstandet.

Erfindungsgemäß ist neben der ersten Scannereinheit, die einen Punktscanner aufweist, mindestens eine zweite Scannereinheit vorgesehen, die mindestens einen Linienscanner aufweist, wobei durch beide Scannereinheiten eine Höhe mindestens eines Steins erfassbar ist. In einem Beispiel sind jedoch mindestens zwei Punktscanner vorgesehen. Durch die Kombination aus mindestens zwei Punktscannern und mindestens einem Linienscanner ist es möglich, eine Förderfläche der Produktionsplatte zu erfassen und somit die exakte Höhe von allen Steinen zu messen, die in mehreren Reihen auf einer Produktionsplatte angeordnet sind.

Erfindungsgemäß weist die Steinanalysevorrichtung auch einen Rahmen auf, an dem die Scannereinheiten angeordnet sind. Dabei kann es sich um einen vollständig geschlossenen Rahmen handeln, es wäre jedoch auch möglich, dass dieser Rahmen einen oder auch mehrere geöffnete Bereiche aufweist. Erfindungsgemäß ist die erste und bevorzugt auch die zweite Scannereinheit an einem Querprofil des Rahmens angeordnet, das sich in Querrichtung erstreckt, wobei bevorzugt die Position der ersten und/oder zweiten Scannereinheit in Querrichtung und/oder Vertikalrichtung veränderbar ist. Vorzugsweise ist die Position der zweiten Scannereinheit in Querrichtung und/oder Vertikalrichtung und/oder Längsrichtung veränderbar. Vorzugsweise ist die erste Scannereinheit an einem Wagen angeordnet, der verschiebbar an dem Querprofil angeordnet ist. Besonders bevorzugt weist der Wagen mindestens einen Magneten auf. Es ist auch denkbar, dass die zweite Scannereinheit unterhalb einer Deckplatte und/oder an einem anderen Wagen angeordnet ist, welcher am Rahmen verschiebbar angeordnet ist. Dadurch, dass die Positionen der Scannereinheiten veränderbar sind, lassen sie sich entsprechend den Anforderungen für unterschiedliche Produktionschargen anpassen.

Erfindungsgemäß ist der Linienscanner als Laserscanner ausgebildet und bevorzugt ist der Punktscanner als Laserscanner ausgebildet. Dabei arbeitet der Punktscanner bevorzugt mit einer Frequenz zwischen 1000 Hz und 15000 Hz, bevorzugt zwischen 2000 und 10000 Hz, besonders bevorzugt zwischen 2500 und 7500 Hz.

Die Linie des Linienscanners wird bevorzugt durch eine Optik erzeugt, welche einen Laserstrahl aufweitet. Dabei erstreckt sich die Linie erfindungsgemäß in Querrichtung über die Förderfläche und insbesondere senkrecht zu der oben genannten Längsrichtung.

Der Linienscanner arbeitet dabei bevorzugt mit einer Frequenz zwischen 1000 Hz und 50000 Hz, bevorzugt zwischen 2000 und 30000 Hz, besonders bevorzugt zwischen 2500 und 10000 Hz. Ein Höhenprofilbild der Steine wird vorzugsweise durch eine Aufnahme von Bildzeilen aufgenommen, welche der Linienscanner erfasst. Laserscanner haben eine besonders hohe Präzision, sodass die Steine präzise vermessen werden können. Zudem lässt sich die Größe der Laserstrahlen sehr präzise einstellen.

Es ist weiterhin vorteilhaft, wenn die erste und/oder zweite Scannereinheit mindestens einen Lichtsensor aufweisen. Durch einen Lichtsensor lässt sich der abgegebene Laserstrahl beziehungsweise der vom Stein, oder der Förderfläche reflektierte Laserstrahl erfassen. Vorzugsweise ist der Lichtsensor des Punktscanners und/oder Linienscanners als CCD und/oder CMOS Sensor ausgebildet. Vorzugsweise erfasst der Sensor des Linienscanners zwischen 1000 und 4000 Punkte, bevorzugt zwischen 1000 und 2000 Punkte, besonders bevorzugt zwischen 1200 und 1700 Punkte. Abhängig von der Höhe der Steine und/oder dem Abstand des Linienscanners zu den Steinen kann so eine Auflösung von ca. 1500 Höhenwerten pro Bildzeile zur Analyse der Steine erreicht werden.

Vorteilhaft ist es, wenn der Linienscanner als 3D-Linienscan-Kamera ausgebildet ist. Erfindungsgemäß ist der Linienscanner in der Lage einen Abstand zu einer Oberfläche mindestens eines Steins und bevorzugt in der Lage einen Abstand zu einer anderen Oberfläche, beispielsweise der Oberfläche der Produktionsplatte, durch ein Triangulations-, Laufzeit-, und/oder Lichtschnitt-Verfahren zu messen.

Gemäß einem Beispiel ist auch der Punktscanner in der Lage einen Abstand zu einer Oberfläche mindestens eines Steins zu messen und erfindungsgemäß ist der Punktscanner in der Lage einen Abstand zu einer anderen Oberfläche, beispielsweise der Oberfläche der Produktionsplatte, durch ein Triangulations-, Laufzeit-, und/oder der Lichtschnitt-Verfahren zu messen. Dadurch kann ein absoluter Abstand der Produktionsplatte zu mindestens einer Scannereinheit, sowie der absolute Abstand der Oberfläche mindestens eines Steins zu mindestens einer Scannereinheit erfasst werden. Daneben wäre es auch möglich einen Maximal- oder Minimalabstand zu erfassen oder auch einen Mittelwert zu ermitteln und/oder auszugeben.

Vorzugsweise ist der Punktscanner zwischen 40° und 120°, mehr bevorzugt, zwischen 60 und 100°, besonders bevorzugt um 90° gegenüber dem Linienscanner gedreht angeordnet. Dabei ist der Linienscanner in Längsrichtung und/oder der Punktscanner in Querrichtung zu einer Förderrichtung ausgerichtet.

In einer weiteren Ausführungsform weist die Steinanalysevorrichtung mindestens eine Erfassungseinrichtung zur Erfassung einer Fördergeschwindigkeit der Förderfläche auf. Bevorzugt handelt es sich hierbei um einen Impulsgeber, womit mindestens die zweite Scannereinheit steuerbar ist. Es ist denkbar, dass der Impulsgeber als Kodierer ausgebildet ist.

Der Kodierer erfasst Positionen einer Welle und/oder einer Antriebseinheit und gibt ein Triggersignal aus, wobei die Positionen optisch, magnetisch oder mechanisch erfassbar sind. Vorzugsweise triggert der Impulsgeber den Linienscanner und/oder eine Bildaufnahmeeinrichtung auf Basis des Triggersignals. Daneben kann auch eine etwaige Beschleunigung (oder Verzögerung) gemessen werden. Daraus resultiert der Vorteil, dass die Aufnahme von Bildzeilen und/oder Helligkeitswerten und/oder Farbwerten abhängig der Fördereinrichtung gesteuert werden kann.

Es ist auch denkbar, dass durch die zweite Scannereinheit die Oberfläche des Steins analysierbar ist. Bevorzugt ist durch die zweite Scannereinheit eine Helligkeitsverteilung von zumindest einer der zweiten Scannereinheit zugewandten Oberfläche mindestens eines Steins ermittelbar. Diese Helligkeitsverteilung kann von der Auswerteeinheit ausgewertet, und zu einem Grauwertbild umgewandelt werden. Durch Analyse dieses Grauwertbildes ist es möglich Flecken und/oder unerwünschte Deformationen der Oberfläche der Steine zu identifizieren und als Defekt mittels der Anzeigeeinrichtung sichtbar zu machen und/oder in eine Bewertung von Produktionsergebnissen mit einfließen zu lassen.

Es ist auch vorteilhaft, wenn die Steinanalysevorrichtung mindestens einen Positionssensor aufweist, durch den mindestens eine Position des Punktscanners gegenüber dem Rahmen ermittelbar ist. Bevorzugt ist der Positionssensor als linearer Positionssensor und/oder als Messstab ausgebildet, wobei der Positionssensor die Position mindestens eines Magneten eines Wagens erfasst, an dem mindestens ein Punktscanner angeordnet ist. Mittels der Positionen der Punktscanner und/oder Linienscanner gegenüber dem Rahmen lassen sich auch die Positionen der Punktscanner und Linienscanner im Verhältnis zueinander ermitteln. Dadurch sind die Positionen (bzw. Mittelpunkte) der einzelnen Steine sowie Informationen über Lücken zwischen den Steinen ermittelbar.

Vorzugsweise sind die Scannereinheiten, der Positionssensor und die Auswerteeinheit datentechnisch über Kabel oder kabellos miteinander verbunden, sodass Daten ausgetauscht werden können. Es ist denkbar, dass auch die Steuereinrichtung datentechnisch mit den Scannereinheiten, dem Positionssensor und/oder der Auswerteeinheit verbunden ist. Auf Basis dieser Daten ist die relative Höhe zwischen einer Oberfläche der Produktionsplatte, auch zwischen den Steinen, und der Oberfläche aller Steine auf der Produktionsplatte ermittelbar. Besonders bevorzugt sind die so erfassten Positionen der einzelnen Steine in einer Datenbank speicherbar. Dies hat den Vorteil, dass die tatsächliche Höhe der Steine messbar ist und defekte Steine nach der Analyse aussortiert werden können.

In einer bevorzugten Ausführungsform weist die Steinanalysevorrichtung mindestens eine Bildaufnahmeeinrichtung auf, die zur ortsaufgelösten Erfassung eines Bildes geeignet und bestimmt ist. Vorzugsweise ist die Bildaufnahmeeinrichtung als Farbkamera oder Schwarzweiß-Kamera ausgebildet und/oder weist einen CCD-Sensor, einen CMOS-Sensor und/oder einen anderen Sensor auf, der Helligkeitswerte und/oder Farbwerte erfassen kann. Es ist denkbar, dass die Bildaufnahmeeinrichtung als Zeilenkamera ausgebildet ist und/oder einzelne Bildzeilen erfasst werden, die zu einem Gesamtbild zusammensetzbar sind. Eine Farbkamera ermöglicht eine Farbanalyse mindestens einer der Farbkamera zugewandten Oberfläche mindestens eines Steins. Durch die Farbanalyse ist eine Überprüfung der Einhaltung vorgegebener Farbtöne für einfarbige Oberflächen der Steine und/oder Farbverteilungen bei mehrfarbigen Oberflächen der Steine möglich.

Bevorzugt weist die Steinanalysevorrichtung eine Beleuchtungseinrichtung auf. Die Beleuchtungseinrichtung umfasst bevorzugt LED Leuchtmittel, die eine zeitlich und farblich konstante und gleichmäßige Beleuchtung einem sich in Querrichtung und/oder Längsrichtung erstreckenden, definierten Flächenabschnitt der Förderfläche ermöglichen. Bevorzugt wird eine durch Bildaufnahmeeinrichtung erfassbare Zeile durch die Beleuchtungseinrichtung beleuchtet. Es ist aber auch denkbar, dass die Beleuchtungseinrichtung andere Leuchtmittel, beispielsweise einem auf einem Glühdraht basierendem und/oder Quecksilberdampf basierendem Leuchtmittel aufweist. Der Einsatz einer konstanten und gleichmäßigen Beleuchtung ermöglicht die Erfassung von Farbmustern verschiedener Steinsorten unter gleichen Bedingungen. Eine Abweichung der ermittelten Farbmuster von vorgegeben Farbmustern kann damit optimal erfasst und bewertet werden. Zudem können durch den Einsatz von hochauflösenden Farbkameras und spezieller Beleuchtung Haarrisse in Steinen detektiert werden.

Vorteilhaft ist es, wenn die Steinanalysevorrichtung mindestens eine Leseeinheit aufweist, die zum Auslesen einer Markierung geeignet ist. Idealerweise ist durch die Leseeinheit ein Transpondersignal bzw. Funketikett, ein QR-Code und/oder ein Barcode lesbar. Wenn die Produktionsplatten einen entsprechenden Code oder Funketikett aufweisen, kann dieses Funketikett und/oder dieser Code erfasst werden. Dadurch sind die Produktionsplatten mit den darauf befindlichen Steinen verfolgbar. Auf diese Weise lassen sich defekte Steine über Aussortierroboter nach der Analyse aussortieren. Es ist auch möglich, die Produktionsplatten einmalig vor der Beladung mit Steinen zu vermessen und/oder zu wiegen, um die Eigenschaften der unbeladenen Produktionsplatten bei späteren Auswertungen heranziehen zu können.

In einer weiteren Ausführungsform umfasst die Steinanalysevorrichtung mindestens eine Gewichtsmesseinheit, womit ein Gewicht eines und/oder mehrerer Steine auf der Förderfläche messbar ist. Bevorzugt ist die Gewichtsmesseinheit als Wägezelle ausgebildet. Durch die Gewichtsmesseinheit ist das Gesamtgewicht der Produktionsplatte und/oder der darauf befindlichen Steine ermittelbar. Abweichungen von vorgegeben Gewichten lassen sich auf diese Weise identifizieren, sodass fehlerhaft beladene Produktionsplatten frühzeitig erkannt und/oder von der Fördereinrichtung entfernt werden können.

Es ist denkbar, dass die Steinanalysevorrichtung in der Standardausführung wenigstens zwei und bevorzugt drei Punktlaser und zwei Linienscanner aufweist. Weiterhin ist bevorzugt der Rahmen aus Aluminium, Metall, Kunststoff, Holz und/oder einem Verbundmaterial ausgestaltet. Bevorzugt, weist der Rahmen ein Gestell aus Alu-Profilen und/oder -Leisten auf. Es ist auch vorteilhaft, wenn die Steinanalysevorrichtung einen Schaltschrank aufweist, der die Steuereinrichtung und/oder Auswerteeinrichtung umfasst. Auf diese Weise sind die Steuereinrichtung und/oder Auswerteeinrichtung nahe beieinander angeordnet, sodass dessen Bedienung und/oder Wartung erleichtert wird. Bevorzugt sind die Steuereinrichtung und Auswerteinrichtung datentechnisch, über Kabel oder Funk miteinander verbunden. Idealerweise, ist die Analyse der physikalischen Eigenschaften der Steine automatisiert und/oder die Steuereinrichtung steuert die Bildaufnahmeeinrichtung, die erste Scannereinheit und/oder die zweite Scannereinheit auf Basis eines Signals, welches die Auswerteeinrichtung abgibt. Besonders bevorzugt steuert die Steuereinrichtung auch einen Aussortierroboter, der auf Basis der gespeicherten Positionen der einzelnen Steine fehlerhafte Steine aussortiert.

Die Aufgabe wird auch durch ein Steinanalyseverfahren zur Bewertung von Steinen, insbesondere Betonsteinen, nach Anspruch 10 gelöst. Das Steinanalyseverfahren erfolgt durch eine Steinanalysevorrichtung zur Anordnung an einer Fördereinrichtung zum Fördern der Steine und mit einer Förderfläche zum Auflegen der Steine, welche sich in einer Längsrichtung und einer Querrichtung (die bevorzugt senkrecht zueinander stehen) erstreckt. Die Steinanalysevorrichtung weist weiterhin mindestens eine erste Scannereinheit zum Scannen mindestens einer physikalischen Eigenschaft der Steine, eine Auswerteeinrichtung zum Auswerten eines von der Scannereinheit ausgegebenen Signals, eine Steuereinrichtung und eine Anzeigeeinrichtung auf. Die Scannereinheit ist von der Förderfläche in einer Vertikalrichtung, senkrecht zur Längsrichtung und Querrichtung beabstandet.

Das Steinanalyseverfahren umfasst unter anderem die Schritte:
a. Erfassen eines ersten Abstandes der ersten Scannereinheit zu der Förderfläche,
b. Erfassen eines zweiten Abstandes einer zweiten Scannereinheit zu einer oberen Fläche eines auf der Förderfläche befindlichen Steins,
c. Ermitteln eines dritten Abstandes der Förderfläche relativ zu der oberen Fläche des auf der Förderfläche befindlichen Steins.

In einem ersten Verfahrensschritt wird ein erster Abstand der ersten Scannereinheit zu der Förderfläche beispielsweise der der Scannereinheit zugewandten Oberfläche der Produktionsplatte erfasst. Vorzugsweise überträgt die erste Scannereinheit ein erstes Messsignal an die Auswerteeinheit. Dadurch können kleinere Unebenheiten, wie Risse oder Verschmutzungen auf der Produktionsplatte identifiziert und im Zuge der späteren Datenauswertung gefiltert und wenn nötig automatisch korrigiert werden.

Ein zweiter Verfahrensschritt besteht darin, einen zweiten Abstand einer zweiten Scannereinheit zu einer oberen Fläche eines auf der Förderfläche befindlichen Steins zu erfassen. Bevorzugt überträgt die zweite Scannereinheit ein zweites Messsignal an die Auswerteeinheit.

In einem dritten Verfahrensschritt wird ein dritter Abstand der Förderfläche relativ zu der oberen Fläche des auf der Förderfläche befindlichen Steins ermittelt. Auf diese Weise kann die exakte Höhe eines Steins auf der Produktionsplatte befindlichen Steins gemessen werden.

Es ist vorteilhaft, wenn zwischen Schritt b. und c. eine Position der ersten Scannereinheit relativ zu der zweiten Scannereinheit ermittelt und/oder vorgegeben wird. Dadurch lassen sich die Positionen von Sensoren zur Abstandsmessung erfassen. Es ist denkbar, dass die erste Scannereinheit als Punktscanner ausgebildet, welcher den Abstand der Oberflächen von Steinen zum Punktscanner und den Abstand von der der Scannereinheit zugewandten Oberfläche der Produktionsplatte erfasst. Auf diese Weise können Lücken zwischen Steinen, und damit die Oberfläche der Produktionsplatte zwischen Steinen erfasst werden, sowie die Position von Steinen auf der Produktionsplatte. Dadurch ist auch die Anzahl von Steinen erfassbar, die auf der Produktionsplatte in einer Reihe bezüglich der Längsrichtung angeordnet sind.

Weiterhin ist es denkbar, dass die zweite Scannereinheit als Linienscankamera ausgebildet ist, die durch die Methode der Lasertriangulation, einen absoluten Abstand der Oberfläche der Steine zur zweiten Scannereinheit erfasst.

In einer Ausführungsform ermittelt ein, bevorzugt linearer, Positionssensor die Position der ersten Scannereinheit gegenüber des Rahmens und/oder überträgt ein Positionssignal an die Auswerteeinheit. Auf Basis des Positionssignals und des ersten und/oder zweiten Messsignals ermittelt die Auswerteeinheit die Positionen der Steine auf der Produktionsplatte, die Lücken zwischen den Steinen und/oder den relativen Abstand der Oberfläche jedes Steins zur Oberfläche der Produktionsplatte. Auf diese Weise ist die individuelle Höhe jedes Steins auf der Produktionsplatte messbar.

Es ist auch denkbar, dass auf Basis des ersten Messsignals und/oder eines Impulssignals, welches ein Impulsgeber abgibt, eine Messung der zweiten Scannereinheit und/oder einer Bildaufnahmeeinrichtung gesteuert wird.

Es ist auch vorteilhaft, wenn über die Bildaufnahmeeinrichtung, beispielsweise einer Farbkamera ein Farbmuster und/oder Grauwertmuster der Steine ermittelt und ein Farbsignal an die Auswerteeinheit gesendet wird. Bevorzugt werden die Steine auf Basis des Farbsignals und vorgegebener Farbgrenzwerte automatisch und/oder manuell bewertet.

Ist die Bewertung positiv, wird das ermittelte Farbmuster und/oder Grauwertmuster gespeichert, bevorzugt werden zwischen 100 und 1000, mehr bevorzugt zwischen 150 und 800, besonders bevorzugt zwischen 250 und 500 Farbmuster und/oder Grauwertmuster gespeichert. Vorzugsweise dienen gespeicherte Farbmuster und/oder Grauwertmuster als Basis für Bewertungen von ermittelten Farbmustern und/oder Grauwertmustern der Steine. Dadurch wird die automatische Bewertung kontinuierlich trainiert. Bevorzugt weist daher die oben genannte Vorrichtung eine Speichereinrichtung zum Speichern von Referenzdaten und insbesondere von Bilddaten auf.

In einer Ausführungsform wird während eines Transportes der Produktionsplatte auf Basis des ersten und/oder zweiten Messsignals ein Höhenprofilbild der Produktionsplatte und der darauf befindlichen Steine erzeugt. Bevorzugt wird dieses Höhenprofilbild als Signal an die Anzeigeeinrichtung gesendet und/oder als 3D Punktwolke in der Anzeigeeinrichtung angezeigt. Dadurch können fehlerhafte Steine sichtbar gemacht und bewertet werden.

In einer weiteren Ausführungsform wird ein Gewicht der Produktionsplatte mit darauf befindlichen Steinen und/oder mindestens eines Steins über eine Gewichtsmesseinheit ermittelt. Vorzugsweise sendet die Gewichtsmesseinheit ein Gewichtssignal an die Auswerteeinheit.

Es ist vorteilhaft, wenn auf Basis des ersten Messsignals, zweiten Messsignals, Positionssignals, Farbmusters, Grauwertmusters, Gewichtssignals und/oder gespeicherter Parameter die Steine automatisch und/oder manuell bewertet werden. Vorzugsweise wird eine Steinhöhe zusätzlich auf Basis gespeicherter Parameter bewertet, die vorgegebene Informationen zu einer Sollhöhe des Steins und/oder minimaler und/oder maximaler Toleranz- und/oder Grenzwerte enthalten.

Vorzugsweise wird eine Steinoberfläche zusätzlich auf Basis gespeicherter Parameter bewertet, die vorgegebene Informationen zu Toleranzwerten für eine Tiefe und Fläche einer Vertiefung und/oder Höhe und Fläche einer Erhöhung enthalten. Vorzugsweise wird eine Steinfarbe zusätzlich auf Basis gespeicherter Parameter bewertet, die vorgegebene Informationen zu Toleranzwerten für Farbabweichungen, Flächen gleicher oder ähnlicher Farben und/oder ein Verhältnis von einzelnen Farben eines Steins zueinander enthalten. Vorzugsweise wird ein Gewicht zusätzlich auf Basis gespeicherter Parameter bewertet, die vorgegebene Informationen zu Toleranzwerten für das Gewicht der Steine in Abhängigkeit der ermittelten Steinhöhe enthalten. Durch eine derartige Bewertung können fehlerhafte Steine identifiziert und aussortiert werden. Dabei ist es denkbar, dass das Gewicht der Steine für eine solche Bewertung ausschlaggebend ist.

In einer weiteren Ausführungsform werden das ermittelte Gewicht der leeren Produktionsplatte, der Produktionsplatte mit darauf befindlichen Steinen und/oder mindestens eines Steins, die ermittelte Steinhöhe, das ermittelte Höhenprofilbild, das ermittelte Farbmuster, das ermittelte Grauwertmuster, eine Position eines Steins auf der Produktionsplatte und/oder eine Markierung der Produktionsplatte in einer Datenbank gespeichert. Vorzugsweise ist ein Gewicht einer leeren Produktionsplatte mit einer Markierung derselben Produktionsplatte gekoppelt in der Datenbank gespeichert. Dadurch ist es möglich, ein Leergewicht einer mit Steinen beladenen Produktionsplatte zu ermitteln und dieses Leergewicht vom Gewicht einer mit Steinen beladenen Produktionsplatte abzuziehen und somit ein Gewicht der Steine zu ermitteln. Es ist aber auch denkbar, dass ein Durchschnittsgewicht mehrerer leerer Produktionsplatten manuell über die Steuereinrichtung in der Datenbank gespeichert wird.

Weitere, Ziele, Vorteile, Merkmale und Anwendungsmöglichkeiten der vorliegenden Erfindung ergeben sich aus der nachfolgenden Beschreibung von Ausführungsbeispielen anhand der Zeichnungen. Der Schutzumfang der vorliegenden Erfindung wird allerdings nur durch die anhängigen Ansprüche definiert.

Dabei zeigt:
- Fig. 1: Eine schematische perspektivische Ansicht einer Steinanalysevorrichtung
- Fig. 2: Eine schematische Frontansicht einer Steinanalysevorrichtung
- Fig. 3: Eine schematische Seitenansicht einer Steinanalysevorrichtung
- Fig. 4: Schematische Darstellung eines Verfahrens zur Bewertung von Steinen

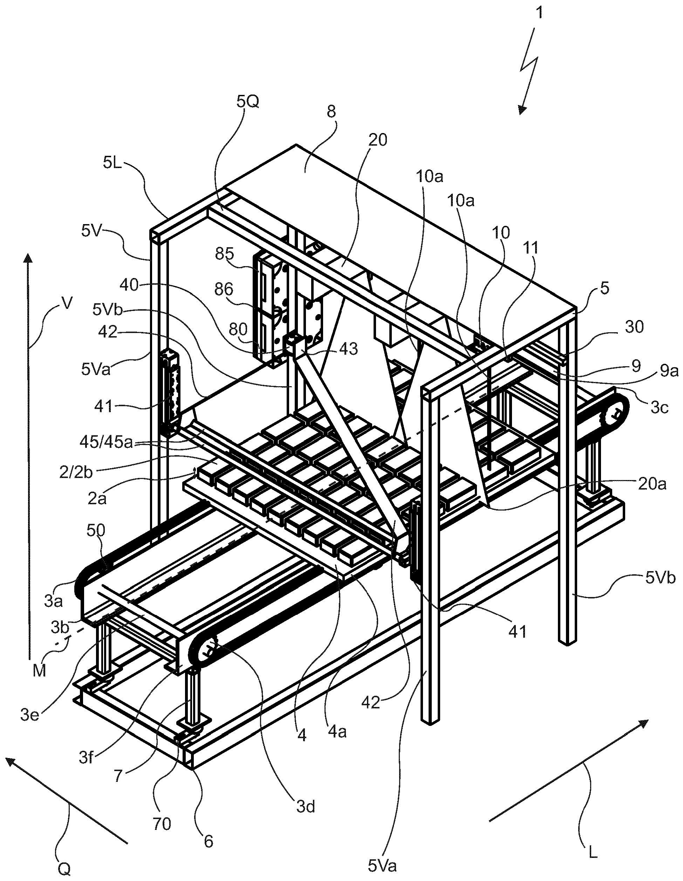

In Figur 1 ist eine Fördereinrichtung 3 und eine Steinanalysevorrichtung 1 dargestellt. Die Steinanalysevorrichtung 1 weist einen Rahmen 5 und mindestens eine erste Scannereinheit 10 auf. Die Steinanalysevorrichtung 1 ist modular aufgebaut, sodass einzelne Elemente, insbesondere Scanner und/oder Sensoren, beweglich bezüglich des Rahmens 5 angeordnet sind. Ebenso ist es möglich zusätzliche Elemente, insbesondere Scanner und/oder Sensoren, nachträglich am Rahmen 5 zu verbringen, oder den Rahmen mit zusätzlichen Rahmenelementen zu erweitern. Auf diese Weise kann die Steinanalysevorrichtung 1 beliebig an die vorgesehene Anwendung angepasst werden.

Die Fördereinrichtung 3 weist einen Sockel 6 und mindestens eine Kettenbahn, bevorzugt aber zwei Kettenbahnen 3a auf, die in Längsrichtung L bewegbar sind, sodass ein Förderelement 4a mit einer Förderfläche 4 in Längsrichtung L durch die Kettenbahnen 3a über die Fördereinrichtung transportierbar ist. An der Fördereinrichtung 3 sind zudem ein Impulsgeber 50 und/oder eine Gewichtsmesseinheit 70 der Steinanalysevorrichtung 1 angeordnet. Es ist auch denkbar, dass die Fördereinrichtung als Fließband oder Gleitbahn ausgebildet ist, oder Keilriemen zum Befördern von Platten aufweist.

Die Kettenbahnen 3a erstrecken sich in Längsrichtung L von einem hinteren Ende 3b zu einem vorderen Ende 3c der Fördereinrichtung 3. Vorzugsweise werden die Kettenbahnen 3a durch Zahnräder 3d angetrieben, die seitlich an der Fördereinrichtung 3 und parallel zu einer sich in Vertikalrichtung V und Querrichtung Q erstreckenden seitlichen Fläche 3f angeordnet sind. An einem Ende 3b, 3c, der Fördereinrichtung 3 sind mindestens 2 Zahnräder angeordnet, die durch eine rotierbare Achse 3e miteinander verbunden sind. Mindestens ein Impulsgeber 50 ist an einem Zahnrad und/oder der rotierbaren Achse angeordnet. Die Fördereinrichtung 3 kontaktiert durch Stützelemente 7 den Sockel 6. Bevorzugt ist zwischen dem Sockel 6 und den Stützelementen 7, zwischen den Stützelementen 7 und der Fördereinrichtung 3 und/oder zwischen der Fördereinrichtung 3 und dem Förderelement 4a mindestens eine Gewichtsmesseinheit 70 angeordnet.

Der Rahmen 5 weist Vertikalelemente 5V, Längselemente 5L, Querelemente 5Q und/oder eine Abdeckplatte 8 auf, welche sich ein Längsrichtung L und Querrichtung Q erstreckt. Bevorzugt sind an den Vertikalelementen 5V auch seitliche Abdeckungen (nicht eingezeichnet) angeordnet, welche sich in Längs- L und Vertikalrichtung V erstrecken. Bevorzugt ist jeweils ein vorderes Vertikalelement 5Va und ein hinteres Vertikalelement 5Vb an Enden eines Längselements 5L angeordnet und jeweils zwei Längselemente 5L an Enden von jeweils einem von zwei Querelementen 5Q. Die Abdeckplatte ist dabei auf mindestens zwei Längselementen 5L und mindestens einem Querelement 5Q angeordnet. Zwischen den zwei vorderen Vertikalelementen 5Va und den zwei hinteren Vertikalelementen 5Vb ist die Fördereinrichtung 3 angeordnet, wobei die vorderen Vertikalelemente 5Va und hinteren Vertikalelemente 5Vb jeweils derart voneinander beabstandet sind, dass durch die Fördereinrichtung 3 ein Förderelement 4a mit einer bevorzugten Breite bezüglich der Querrichtung Q zwischen 100 cm und 300 cm, mehr bevorzugt zwischen 120 cm und 150 cm transportierbar ist.

An den zwei vorderen Vertikalelementen 5Va sind zwei Höhenverstellungseinrichtungen 41 angeordnet, die sich gegenüber stehen, wobei die Höhenverstellungseinrichtungen 41 in Vertikalrichtung V oberhalb der Fördereinrichtung 3 und oberhalb der Förderfläche 4 angeordnet sind. Die Beleuchtungseinrichtung 45 erstreckt sich zwischen den zwei Höhenverstellungseinrichtungen 41 und ist durch die Höhenverstellungseinrichtungen 41 in ihrer Höhe bezüglich der Vertikalrichtung V veränderbar. Dabei sind mindestens zwei Halteelemente 42 an der Beleuchtungseinrichtung 45 und/oder jeweils einer Höhenverstellungseinrichtungen 41 angeordnet, an dessen oberen Enden 43 die Bildaufnahmeeinrichtung 40 verbracht ist. Dabei ist die Bildaufnahmeeinrichtung 40 abhängig, oder unabhängig von der Beleuchtungseinrichtung 45 durch die Höhenverstellungseinrichtungen 41 in ihrer Höhe bezüglich der Vertikalrichtung V veränderbar.

Die Beleuchtungseinrichtung 45 weist zwei Beleuchtungselemente 45a auf, welche sich in Querrichtung erstrecken und bezüglich der Längsrichtung L voneinander beabstandet sind. Weiterhin sind die Beleuchtungselemente 45a parallel zu der Förderfläche 4 ausgerichtet, wobei die Bildaufnahmeeinrichtung 40 derart ausgerichtet ist, dass sie ein Bild von einem Abschnitt der Förderfläche 4 zwischen den zwei Beleuchtungselementen 45a erfasst. Zwischen den zwei hinteren Vertikalelementen 5Vb ist ein Querprofil 9 angeordnet, das sich in Querrichtung Q erstreckt und mindestens eine in Querrichtung Q verlaufende Nut aufweist. An der Nut 9a ist mindestens ein Wagen 11 angeordnet, welcher in Querrichtung verschiebbar ist. Der Wagen 11 weist mindestens eine erste Scannereinheit 10 und einen Magneten auf. Parallel zu dem Querquerprofil ist ein Positionssensor angeordnet, welcher bevorzugt leistenförmig ist. Der Positionssensor 30 erstreckt sich ebenfalls in Querrichtung Q und ist zwischen den zwei hinteren Vertikalelementen 5Vb und/oder oberhalb des Querprofils 9 und/oder oberhalb der ersten Scannereinheit 10 verbracht. Zudem ist der Positionssensor 30 in der Lage den Magneten des Wagens 11 zu detektieren und auf diese Weise die Position der ersten Scannereinheit 10 relativ zum Rahmen 5 zu erfassen.

Erfindungsgemäß weist die Steinanalysevorrichtung 1 drei erste Scannereinheiten 10 auf, die als Punktscanner 10 ausgebildet sind. Die drei Punktscanner 10 sind bevorzugt jeweils an einem Wagen mit jeweils einem Magneten angeordnet. Jeweils ein Punktscanner 10 ist derart ausgerichtet, dass ein Punktscanner 10 bezüglich der Vertikalrichtung V und Querrichtung Q oberhalb jeweils einer Kettenbahn 3a angeordnet ist und ein Laserstrahl 10a senkrecht bezüglich der Längsrichtung L und Querrichtung Q auf die Förderfläche 4 auftrifft. Der dritte Punktscanner 10 ist derart ausgerichtet, dass der Laserstrahl 10a senkrecht bezüglich der Längsrichtung L und Querrichtung Q, bevorzugt an einer Mittellinie M auf die Förderfläche 4 auftrifft. Vorzugsweise sind die Punktscanner zwischen 10 cm und 150 cm, mehr bevorzugt zwischen 30 cm und 100 cm, besonders bevorzugt zwischen 50 cm und 80 cm bezüglich der Vertikalrichtung V oberhalb der Förderfläche 4 angeordnet.

Vorzugsweise weist die Steinanalysevorrichtung 1 zwei zweite Scannereinheiten 20 auf, die als Linienscanner 20, bevorzugt als Laserlinienscanner ausgebildet sind. Dabei erstreckt sich der vom Linienscanner abgegebene Laserstrahl 20b bezüglich der Querrichtung Q und/oder breitet sich fächerartig aus und trifft auf der Förderfläche 4 und/oder der Oberfläche 2b der Steine 2 auf. Die Scanlinie 20a ist parallel zur Querrichtung Q und senkrecht zur Längsrichtung L und Vertikalrichtung V ausgerichtet. Vorzugsweise sind die Linienscanner 20 zwischen 20 cm und 200 cm, mehr bevorzugt zwischen 50 cm und 150 cm, besonders bevorzugt zwischen 65 cm und 100 cm bezüglich der Vertikalrichtung V oberhalb der Förderfläche 4 angeordnet. Idealerweise sind die Linienscanner 20 an und/oder mindestens eines Querelements 5Q angeordnet.

An einem Vertikalelement 5V, Querelement 5Q und/oder Längselement ist ein Schaltschrank 86 angeordnet. Der Schaltschrank 86 weist eine Auswerteeinrichtung 80 und/oder eine Steuereinrichtung 85 auf.

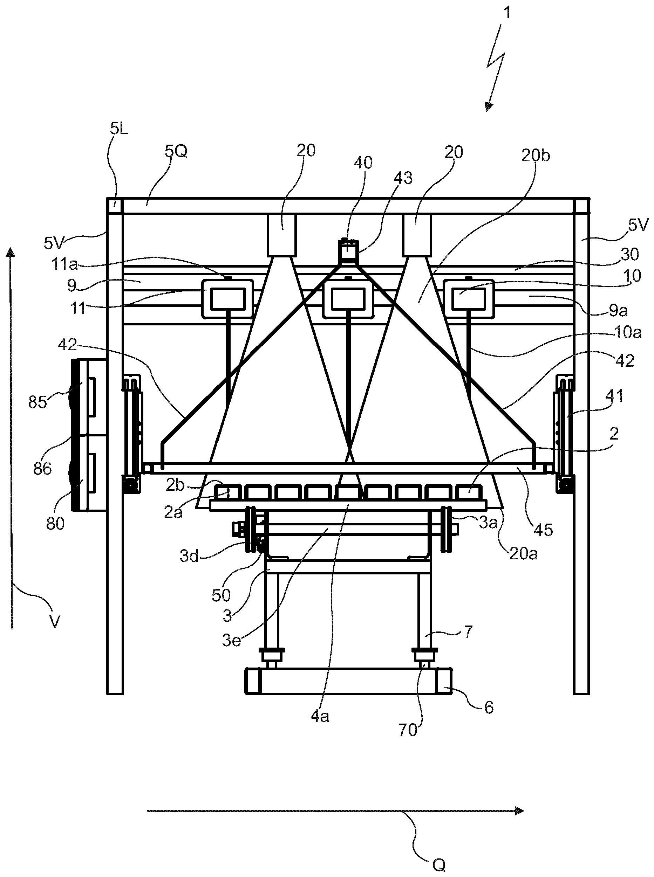

In der Figur 2 ist die Steinanalysevorrichtung 1 in einer Frontansicht dargestellt. Die drei Punktscanner 10 sind an den verschiebbaren Wagen 11 mit den Magneten 11a an der Nut 9a des Querprofils 9 angeordnet. Dabei sind die Punktscanner 10 bezüglich der Querrichtung Q nebeneinander und voneinander beabstandet angeordnet. Der Positionssensor 30 ist parallel zum Querprofil 9 angeordnet. Bezüglich der Vertikalrichtung V sind die Linienscanner 20 in einem größeren Abstand zur Förderfläche 4 angeordnet, als die Punktscanner 10. Die Förderfläche mit den darauf befindlichen Steinen 2 ist bezüglich der Vertikalrichtung V unterhalb der Punktscanner 10, der Linienscanner 20, der Beleuchtungseinrichtung 45 und/oder der Bildaufnahmeeinrichtung 40 angeordnet. Dadurch ist die Höhe der Steine 2a optimal durch die Punktscanner 10 und/oder die Linienscanner 20 messbar.

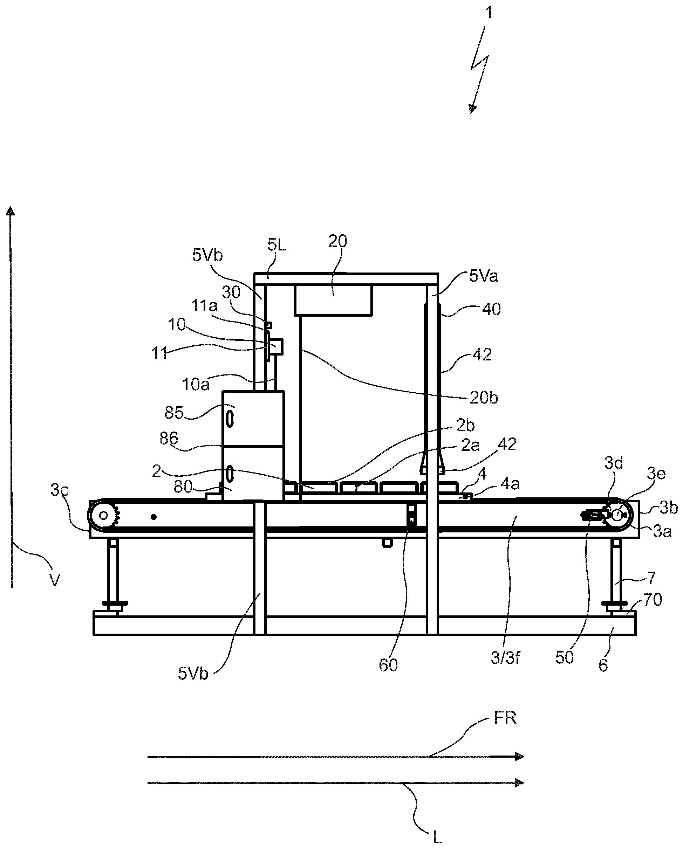

In der Figur 3 ist die Steinanalysevorrichtung 1 in einer Seitenansicht dargestellt. Das Förderelement 4a mit den darauf befindlichen Steinen 2 wird in Förderrichtung FR über die Fördereinrichtung 3 und die Kettenbahnen befördert. Die Messeinrichtungen sind bezüglich der Längsrichtung L hintereinander angeordnet, sodass der Impulsgeber 50 am hinteren Ende 3b der Fördereinrichtung 3 angeordnet ist. Die Beleuchtungseinrichtung 45 und die Bildaufnahmeeinrichtung 40 sind näher an dem hinteren Ende 3b angeordnet, als am vorderen Ende 3c. Die Punktscanner 10 sind bezüglich der Längsrichtung L näher am vorderen Ende 3c angeordnet, als am hinteren Ende 3b. Die Linienscanner 20 sind bezüglich der Längsrichtung L zwischen den Punktscannern 10 und der Bildaufnahmeeinrichtung 40 angeordnet. Dabei ist eine Leseeinheit bezüglich der Vertikalrichtung V unterhalb des Förderelements 4a, an der Fördereinrichtung 3 angeordnet und/oder bezüglich der Längsrichtung L bevorzugt zwischen dem hinteren Ende 3b und der Beleuchtungseinrichtung 45, oder zwischen der Beleuchtungseinrichtung 45 und dem Linienscanner 20 und/oder dem Punktscanner 10.

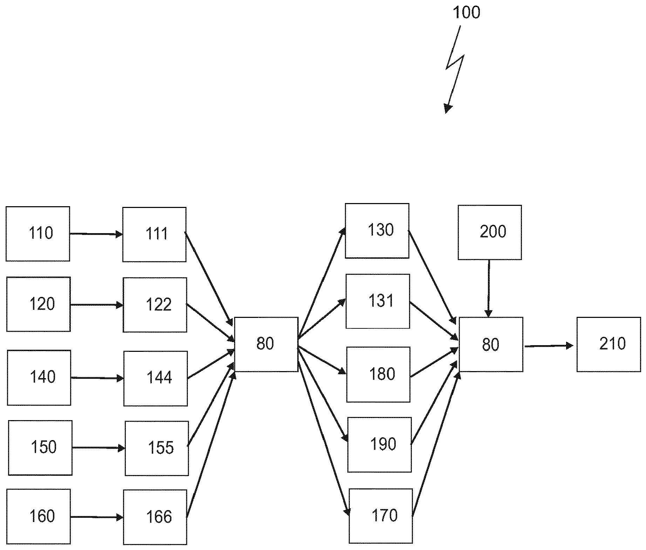

In Figur 4 ist ein Steinanalyseverfahren 100 zur Bewertung 210 von Steinen 2 dargestellt. In einem ersten Verfahrensschritt wird ein erster Abstand 110 der ersten Scannereinheit 10 zu der Förderfläche 4 erfasst. Vorzugsweise überträgt die erste Scannereinheit 10 auf Basis des ersten Abstands 110 ein erstes Messsignal 111 an die Auswerteeinheit 80.

Ein zweiter Verfahrensschritt besteht darin, einen zweiten Abstand 120 einer zweiten Scannereinheit 20 zu einer oberen Fläche 2b eines auf der Förderfläche 4 befindlichen Steins 2 zu erfassen. Bevorzugt überträgt die zweite Scannereinheit 20 auf Basis des zweiten Abstands 120 ein zweites Messsignal 122 an die Auswerteeinheit 80.

Ein vorteilhafter Verfahrensschritt sieht vor, durch den Positionssensor 30 eine Position 140 eines Magneten eines Wagens erfasst, an dem die erste Scannereinheit angeordnet ist, also eine Position 140 der ersten Scannereinheit 10 gegenüber des Rahmens 5 zu erfassen. Bevorzugt überträgt der Positionssensor 30 auf Basis der Position 140 ein Positionssignal 144 an die Auswerteeinheit 80.

Ein vorteilhafter Verfahrensschritt sieht vor, dass über die Bildaufnahmeeinrichtung 40 ein Farbmuster 150 der Steine ermittelt und auf Basis dessen ein Farbsignal 155 an die Auswerteeinheit 80 gesendet wird.

Bevorzugt wird auch ein Gewicht 160 des Förderelements 4a mit darauf befindlichen Steinen 2 ermittelt und auf Basis dessen ein Gewichtssignal 166 von der Gewichtsmesseinheit 70 an die Auswerteeinheit 80 gesendet.

In einem weiteren Verfahrensschritt werden das erste Messsignal 111, das zweite Messsignal 122, das Positionssignal 144, das Farbsignal 155 und/oder Gewichtssignal 166 in der Auswerteeinheit 80 ausgewertet. Auf Basis des ersten Messsignals 111, des zweiten Messsignals 122, das Positionssignals 144, das Farbsignals 155 und/oder Gewichtssignals 166 werden eine Steinhöhe 2a beziehungsweise ein dritter Abstand 130 der Förderfläche 4 relativ zu der oberen Fläche 2b des auf der Förderfläche 4 befindlichen Steins 2, ein Höhenprofilbild 131, eine Steinoberfläche 170, eine Steinfarbe 180 und/oder ein Steingewicht 130 ermittelt.

Nach der Ermittlung der Eigenschaften der Steine 2 werden zur Bewertung 210 der Steine 2 gespeicherte Parameter 200 herangezogen. Vorzugsweise weisen die gespeicherten Parameter 200 Vorgabewerte auf, die zusammen mit der Steinhöhe 2a, dem Höhenprofilbild 131, der Steinoberfläche 170, der Steinfarbe 180 und/oder dem Steingewicht 130 eine Basis für die Bewertung 210 bilden. Bevorzugt weisen die Vorgabewerte beziehungsweise gespeicherten Parameter 200 Informationen zu Toleranzwerten für eine Tiefe und Fläche einer Vertiefung und/oder Höhe und Fläche einer Erhöhung einer Steinoberfläche auf, sowie Informationen zu Toleranzwerten für Farbabweichungen, Flächen gleicher oder ähnlicher Farben und/oder ein Verhältnis von einzelnen Farben eines Steins zueinander, sowie Informationen zu Toleranzwerten für ein Steingewicht 190 in Abhängigkeit der ermittelten Steinhöhe 2a.

### Bezugszeichenliste

- 1.: Steinanalysevorrichtung
- 2.: Stein / Steine
- 2a.: Höhe eines Steins
- 2b.: Oberfläche eines Steins
- 3.: Fördereinrichtung
- 3a.: Kettenbahnen
- 3b.: hinteres Ende
- 3c.: vorderes Ende
- 3d.: Zahnräder
- 3e.: rotierbare Achse
- 3f.: seitliche Fläche
- 4.: Förderfläche
- 4a.: Förderelement
- 5.: Rahmen
- 5L.: Längselemente
- 5Q.: Querelemente
- 5V.: Vertikalelemente
- 5Va.: vordere Vertikalelemente
- 5Vb.: hintere Vertikalelemente
- 6.: Sockel
- 7.: Stützelemente
- 8.: Abdeckplatte
- 9.: Querprofil
- 9a.: Nut
- 10.: erste Scannereinheit / Punktscanner
- 10a.: Laserstrahl
- 11.: Wagen
- 11a.: Magnet
- 20.: zweite Scannereinheit / Linienscanner
- 20a.: Scanlinie
- 20b.: Laserstrahl des Linienscanners
- 30.: Positionssensor
- 40.: Bildaufnahmeeinrichtung
- 41.: Höhenverstellungseinrichtung
- 42.: Halteelemente
- 43.: oberes Ende
- 45.: Beleuchtungseinrichtung
- 45a.: Beleuchtungselement
- 50.: Impulsgeber / Kodierer
- 60.: Leseeinheit
- 70.: Gewichtsmesseinheit
- 80.: Auswerteeinrichtung
- 85.: Steuereinrichtung
- 86.: Schaltschrank

- 100.: Steinanalyseverfahren
- 110.: erster Abstand
- 111.: erstes Messsignal
- 120.: zweiter Abstand
- 122.: zweites Messsignal
- 130.: dritter Abstand
- 131.: Höhenprofilbild
- 140.: Positionen der ersten Scannereinheit
- 144.: Positionssignal
- 150.: Farbmuster
- 155.: Farbsignal
- 160.: Gewicht
- 166.: Gewichtssignal
- 170.: Steinoberfläche
- 180.: Steinfarbe
- 190.: Steingewicht
- 200.: gespeicherte Parameter
- 210.: Bewertung

- L.: Längsrichtung
- Q.: Querrichtung
- V.: Vertikalrichtung
- FR.: Förderrichtung
- M.: Mittellinie

## Patentansprüche

1. Steinanalysevorrichtung (1) zur Bewertung von Steinen (2), insbesondere Betonsteinen, angeordnet an einer Fördereinrichtung (3) zum Fördern der Steine (2) mit einer Förderfläche (4) zum Auflegen der Steine (2), welche sich in eine Längsrichtung (L) und eine Querrichtung (Q) erstreckt, wobei die Steinanalysevorrichtung (1) drei erste Scannereinheiten (10) zum Scannen mindestens einer physikalischen Eigenschaft der Steine (2), eine Auswerteeinrichtung (80) zum Auswerten der von den Scannereinheiten (10) ausgegebenen Signale, eine Steuereinrichtung (85) und eine Anzeigeeinrichtung aufweist, wobei die Scannereinheiten (10) von der Förderfläche (4) in einer Vertikalrichtung (V), senkrecht zur Längsrichtung (L) und Querrichtung (Q) beabstandet sind,
**wobei**
neben den ersten Scannereinheiten (10), die jeweils einen Punktscanner (10) zur Messung eines ersten Abstands zu der Förderfläche (4), beispielsweise einer der ersten Scannereinheit (10) zugewandten Oberfläche einer Produktionsplatte aufweisen, mindestens eine zweite Scannereinheit (20) vorgesehen ist, die mindestens einen Linienscanner (20) zur Messung eines zweiten Abstands zu einer oberen Fläche (2b) eines auf der Förderfläche (4) befindlichen Steins (2) aufweist, wobei durch beide Scannereinheiten (10, 20) eine Höhe (2a) des mindestens einen Steins (2) erfassbar ist indem ein dritter Abstand der Förderfläche (4) relativ zu der oberen Fläche (2b) des auf der Förderfläche (4) befindlichen Steins (2) ermittelt wird, wobei der Linienscanner (20) als Laserscannner ausgebildet ist und sich eine Linie des Linienscanners in Querrichtung (Q) über die Förderfläche (4) erstreckt, wobei die Steinanalysevorrichtung (1) einen Rahmen (5) aufweist, an dem die ersten und zweiten Scannereinheiten (10, 20) angeordnet sind, wobei die drei ersten Scannereinheiten (10) bezüglich der Querrichtung (Q) nebeneinander und voneinander beabstandet an einem Querprofil (9) des Rahmens (5), das sich in Querrichtung (Q) erstreckt, angeordnet sind.

2. Steinanalysevorrichtung (1) nach Anspruch 1
**dadurch gekennzeichnet, dass**
der Punktscanner (10) als Laserscannner ausgebildet ist.

3. Steinanalysevorrichtung (1) nach einem der vorhergehenden Ansprüche
**dadurch gekennzeichnet, dass**
die erste und/oder zweite Scannereinheit (10), (20) mindestens einen Lichtsensor aufweisen.

4. Steinanalysevorrichtung (1) nach einem der vorhergehenden Ansprüche
**dadurch gekennzeichnet, dass**
die Steinanalysevorrichtung (1) mindestens eine Geschwindigkeitserfassungseinrichtung und insbesondere mindestens einen Impulsgeber (50) umfasst, womit mindestens die zweite Scannereinheit (20) steuerbar ist.

5. Steinanalysevorrichtung (1) nach einem der vorhergehenden Ansprüche
**dadurch gekennzeichnet, dass**
durch die zweite Scannereinheit (20) die Oberfläche des Steins (2b) analysierbar ist.

6. Steinanalysevorrichtung nach einem der vorhergehenden Ansprüche
**dadurch gekennzeichnet, dass**
die Steinanalysevorrichtung (1) mindestens einen Positionssensor (30) aufweist, durch den eine Position des Punktscanners (10) gegenüber eines Rahmens (5) ermittelbar ist.

7. Steinanalysevorrichtung nach einem der vorhergehenden Ansprüche
**dadurch gekennzeichnet, dass**
die Steinanalysevorrichtung (1) mindestens eine Bildaufnahmeeinrichtung (40), die zur ortsaufgelösten Erfassung eines Bildes geeignet und bestimmt ist und/oder eine Beleuchtungseinrichtung (45) aufweist.

8. Steinanalysevorrichtung nach einem der vorhergehenden Ansprüche
**dadurch gekennzeichnet, dass**
die Steinanalysevorrichtung (1) mindestens eine Leseeinheit (60) aufweist, die zum Auslesen einer Markierung geeignet ist.

9. Steinanalysevorrichtung nach einem der vorhergehenden Ansprüche
**dadurch gekennzeichnet, dass**
die Steinanalysevorrichtung (1) mindestens eine Gewichtsmesseinheit (70) aufweist, womit ein Gewicht eines und/oder mehrerer Steine (2) auf der Förderfläche (4) messbar ist.

10. Steinanalyseverfahren (100) zur Bewertung von Steinen (2), insbesondere Betonsteinen, durch eine Steinanalysevorrichtung (1) angeordnet an einer Fördereinrichtung (3) zum Fördern der Steine (2) mit einer Förderfläche (4) zum Auflegen der Steine (2), welche sich in eine Längsrichtung (L) und eine Querrichtung (Q) erstreckt, wobei die Steinanalysevorrichtung (1) drei erste Scannereinheiten (10) zum Scannen mindestens einer physikalischen Eigenschaft der Steine (2), eine Auswerteeinrichtung (80) zum Auswerten der von den Scannereinheiten ausgegebenen Signale, eine Steuereinrichtung (85) und eine Anzeigeeinrichtung aufweist, wobei die Scannereinheiten (10) von der Förderfläche in einer Vertikalrichtung (V), senkrecht zur Längsrichtung (L) und Querrichtung (Q) beabstandet sind,
**umfassend die Schritte:**
a. Erfassen der ersten Abstände (110) der ersten Scannereinheiten (10) zu der Förderfläche (4), wobei die ersten Scannereinheiten (10) als Punktscanner (10) ausgebildet sind, wobei die Steinanalysevorrichtung (1) einen Rahmen (5) aufweist, an dem die ersten Scannereinheiten (10) und eine zweite Scannereinheit (20) angeordnet sind, wobei die drei ersten Scannereinheiten (10) bezüglich der Querrichtung (Q) nebeneinander und voneinander beabstandet an einem Querprofil (9) des Rahmens (5), das sich in Querrichtung erstreckt, angeordnet sind,
b. Erfassen eines zweiten Abstandes (120) der zweiten Scannereinheit (20) zu einer oberen Fläche (2b) eines auf der Förderfläche (4) befindlichen Steins (2), wobei die zweite Scannereinheit (20) einen Linienscanner (20) aufweist und als Laserscannner ausgebildet ist, wobei sich eine Linie des Linienscanners (20) in Querrichtung über die Förderfläche (4) erstreckt,
c. Erfassen einer Höhe (2a) des mindestens einen Steins (2) durch beide Scannereinheiten (10, 20) durch ermitteln eines dritten Abstandes (2a) der Förderfläche (4) relativ zu der oberen Fläche (2b) des auf der Förderfläche (4) befindlichen Steins (2).

11. Steinanalyseverfahren nach Anspruch (10)
**dadurch gekennzeichnet, dass**
eine Position der ersten Scannereinheit (10) relativ zu der zweiten Scannereinheit (20) ermittelt und/oder vorgegeben wird.

## Claims

1. Stone analysis device (1) for the evaluation of stone blocks (2), in particular concrete blocks, arranged on a conveyor device (3) for conveying the stone blocks (2) with a conveying surface (4) on which the stone blocks (2) are laid and which extends in a longitudinal direction (L) and in a transverse direction (Q), wherein the stone analysis device (1) comprises three first scanner units (10) for scanning at least one physical characteristic of the stone blocks (2), an evaluation device (80) for evaluating the signals output by the scanner units, a control device (85) and a display device, wherein the scanner units (10) are spaced apart from the conveying surface (4) in a vertical direction (V), perpendicular to the longitudinal direction (L) and transverse direction (Q),
wherein
in addition to the first scanner units (10) which each comprise a point scanner (10) for measuring a first distance from the conveying surface (4), for example a surface of a production plate facing the first scanner unit (10), at least one second scanner unit (20) is provided, which comprises at least one line scanner (20) for measuring a second distance from an upper surface (2b) of a stone block (2) located on the conveying surface (4), wherein a height (2a) of the at least one stone block (2) can be detected by both scanner units (10, 20) by determining a third distance of the conveying surface (4) relative to the upper surface (2b) of the stone block (2) located on the conveying surface (4), wherein the line scanner (20) is designed as a laser scanner and a line of the line scanner extends in the transverse direction (Q) over the conveying surface (4), wherein the stone analysis device (1) has a frame (5) on which the first and second scanner units (10, 20) are arranged, wherein the three first scanner units (10) are arranged adjacent to one another, with respect to the transverse direction (Q) and spaced apart from one another on a transverse profile (9) of the frame (5) which extends in the transverse direction (Q).

2. Stone analysis device (1) according to claim 1,
**characterised in that**
the point scanner (10) is designed as a laser scanner.

3. Stone analysis device (1) according to any of the preceding claims,
**characterised in that**
the first and/or the second scanner unit (10), (20) comprise(s) at least one light sensor.

4. Stone analysis device (1) according to any of the preceding claims,
**characterised in that**
the stone analysis device (1) comprises at least one speed detection device and, in particular, at least one pulse generator (50) by which at least the second scanner unit (20) is controllable.

5. Stone analysis device (1) according to any of the preceding claims,
**characterised in that**
the surface of the stone block (2b) can be analysed by the second scanner unit (20).

6. Stone analysis device according to any of the preceding claims,
**characterised in that**
the stone block analysis device (1) comprises at least one position sensor (30) by which a position of the point scanner (10) with respect to a frame (5) can be determined.

7. Stone analysis device according to any of the preceding claims,
**characterised in that**
the stone analysis device (1) comprises at least one image recording device (40), which is suitable and intended for the spatially resolved capture of an image and/or has a lighting device (45).

8. Stone analysis device according to any of the preceding claims,
**characterised in that**
the stone analysis device (1) comprises at least one reading unit (60) which is suitable for reading a marking.

9. Stone analysis device according to any of the preceding claims,
**characterised in that**
the stone analysis device (1) comprises at least one weight measurement unit (70), by which a weight of one and/or a plurality of stone blocks (2) on the conveying surface (4) can be measured.

10. Stone analysis method (100) for the evaluation of stone blocks (2), in particular concrete blocks, by a stone analysis device (1), arranged on a conveying device (3) for conveying the stone blocks (2) with a conveying surface (4), on which the stone blocks (2) are laid and which extends in a longitudinal direction (L) and in a transverse direction (Q), wherein the stone analysis device (1) comprises three first scanner units (10) for scanning at least one physical characteristic of the stone blocks (2), an evaluation device (80) for the evaluation of a signal output by the scanner units, a control device (85) and a display device, wherein the scanner units (10) are spaced apart from the conveying surface in a vertical direction (V), perpendicular to the longitudinal direction (L) and transverse direction (Q),
comprising the steps:
a. detecting a first distance (110) of the first scanner unit (10) from the conveying surface (4), wherein the first scanner units (10) are designed as point scanners (10), wherein the stone analysis device (1) has a frame (5) on which the first scanner units (10) and a second scanner unit (20) are arranged, wherein the three first scanner units (10) are arranged adjacent to one another with respect to the transverse direction (Q) and spaced apart from one another on a transverse profile (9) of the frame (5) which extends in the transverse direction,
b. detecting a second distance (120) of the second scanner unit (20) from an upper surface (2b) of a stone block (2) located on the conveying surface (4), wherein the second scanner unit (20) has a line scanner (20) and is designed as a laser scanner, wherein a line of the line scanner (20) extends in the transverse direction over the conveying surface (4),
c. detecting a height (2a) of the at least one stone block (2) by both scanner units (10, 20) by determining a third distance (2a) of the conveying surface (4), relative to the upper surface (2b) of the stone block (2) located on the conveying surface (4).

11. Stone analysis method according to claim 10,
**characterised in that**
a position of the first scanner unit (10) relative to the second scanner unit (20) is determined and/or predefined.

## Revendications

1. Dispositif d'analyse de pierre (1) pour évaluer des pierres (2), en particulier des blocs de béton, agencé au niveau d'un équipement d'acheminement (3) pour acheminer les pierres (2) avec une surface d'acheminement (4) afin de déposer les pierres (2), laquelle s'étend dans une direction longitudinale (L) et une direction transversale (Q), dans lequel le dispositif d'analyse de pierre (1) présente trois premières unités de balayage (10) pour balayer au moins une propriété physique des pierres (2), un équipement d'évaluation (80) pour évaluer les signaux émis par les unités de balayage (10), un équipement de commande (85) et un équipement d'affichage, dans lequel les unités de balayage (10) sont espacées de la surface d'acheminement (4) dans une direction verticale (V), perpendiculaire aux direction longitudinale (L) et direction transversale (Q),
dans lequel
à côté des premières unités de balayage (10), qui présentent respectivement un scanner de points (10) pour mesurer une première distance jusqu'à la surface d'acheminement (4), par exemple une surface, tournée vers la première unité de balayage (10), d'une plaque de production, est prévue au moins une deuxième unité de balayage (20), qui présente au moins un scanner de lignes (20) pour mesurer une deuxième distance jusqu'à une surface supérieure (2b) d'une pierre (2) se trouvant sur la surface d'acheminement (4), dans lequel par les deux unités de balayage (10, 20) une hauteur (2a) de la au moins une pierre (2) peut être saisie en ce qu'une troisième distance de la surface d'acheminement (4) par rapport à la surface supérieure (2b) de la pierre (2) se trouvant sur la surface d'acheminement (4) est déterminée, dans lequel le scanner de lignes (20) est conçu en tant que scanner laser et une ligne du scanner de lignes (20) s'étend dans la direction transversale (Q) par-dessus la surface d'acheminement (4), dans lequel le dispositif d'analyse de pierre (1) présente un cadre (5) au niveau duquel sont agencées les premières et deuxièmes unités de balayage (10, 20), dans lequel les trois premières unités de balayage (10) sont agencées de façon espacée les unes à côté des autres et les unes des autres par rapport à la direction transversale (Q) au niveau d'un profil transversal (9) du cadre (5), lequel profil s'étend dans la direction transversale (Q).

2. Dispositif d'analyse de pierre (1) selon la revendication 1 **caractérisé en ce que**
le scanner de points (10) est conçu en tant que scanner laser.

3. Dispositif d'analyse de pierre (1) selon l'une des revendications précédentes
**caractérisé en ce que**
la première et/ou la deuxième unité de balayage (10), (20) présentent au moins un capteur de lumière.

4. Dispositif d'analyse de pierre (1) selon l'une des revendications précédentes
**caractérisé en ce que**
le dispositif d'analyse de pierre (1) comprend au moins un équipement de saisie de vitesse et en particulier au moins un générateur d'impulsions (50) avec lesquels au moins la deuxième unité de balayage (20) peut être commandée.

5. Dispositif d'analyse de pierre (1) selon l'une des revendications précédentes
**caractérisé en ce que**
la surface de la pierre (2b) peut être analysée par la deuxième unité de balayage (20).

6. Dispositif d'analyse de pierre selon l'une des revendications précédentes
**caractérisé en ce que**
le dispositif d'analyse de pierre (1) présente au moins un capteur de position (30) par lequel une position du scanner de points (10) par rapport à un cadre (5) peut être établie.

7. Dispositif d'analyse de pierre selon l'une des revendications précédentes
**caractérisé en ce que**
le dispositif d'analyse de pierre (1) présente au moins un équipement de prise de vue (40), qui est approprié et déterminé pour saisir avec une résolution spatiale une image, et/ou un équipement d'éclairage (45).

8. Dispositif d'analyse de pierre selon l'une des revendications précédentes
**caractérisé en ce que**
le dispositif d'analyse de pierre (1) présente au moins une unité de lecture (60) qui est appropriée pour lire un marquage.

9. Dispositif d'analyse de pierre selon l'une des revendications précédentes
**caractérisé en ce que**
le dispositif d'analyse de pierre (1) présente au moins une unité de mesure de poids (70) avec laquelle un poids d'une et/ou plusieurs pierres (2) peut être mesuré sur la surface d'acheminement (4).

10. Procédé d'analyse de pierre (100) destiné à évaluer des pierres (2), en particulier des blocs de béton, par un dispositif d'analyse de pierre (1) agencé au niveau d'un équipement d'acheminement (3) pour acheminer les pierres (2) avec une surface d'acheminement (4) afin de déposer les pierres (2), laquelle s'étend dans une direction longitudinale (L) et une direction transversale (Q), dans lequel le dispositif d'analyse de pierre (1) présente trois premières unités de balayage (10) pour balayer au moins une propriété physique des pierres (2), un équipement d'évaluation (80) pour évaluer les signaux émis par les unités de balayage, un équipement de commande (85) et un équipement d'affichage, dans lequel les unités de balayage (10) sont espacées de la surface d'acheminement dans une direction verticale (V), perpendiculaire aux direction longitudinale (L) et direction transversale (Q),
comprenant les étapes consistant à :
a. saisir les premières distances (110) des premières unités de balayage (10) jusqu'à la surface d'acheminement (4), dans lequel les premières unités de balayage (10) sont conçues en tant que scanners de points (10), dans lequel le dispositif d'analyse de pierre (1) présente un cadre (5) au niveau duquel sont agencées les premières unités de balayage (10) et une deuxième unité de balayage (20), dans lequel les trois premières unités de balayage (10) sont agencées de façon espacée les unes à côté des autres et les unes des autres par rapport à la direction transversale (Q) au niveau d'un profil transversal (9) du cadre (5), lequel profil s'étend dans la direction transversale,
b. saisir une deuxième distance (120) de la deuxième unité de balayage (20) jusqu'à une surface supérieure (2b) d'une pierre (2) se trouvant sur la surface d'acheminement (4), dans lequel la deuxième unité de balayage (20) présente un scanner de lignes (20) et est conçue en tant que scanner laser, dans lequel une ligne du scanner de lignes (20) s'étend dans la direction transversale par-dessus la surface d'acheminement (4),
c. saisir une hauteur (2a) de la au moins une pierre (2) par les deux unités de balayage (10, 20) en établissant une troisième distance (2a) de la surface d'acheminement (4) par rapport à la surface supérieure (2b) de la pierre (2) se trouvant sur la surface d'acheminement (4).

11. Procédé d'analyse de pierre selon la revendication 10
**caractérisé en ce que**
une position de la première unité de balayage (10) par rapport à la deuxième unité de balayage (20) est établie et/ou prédéfinie.
